# EUROPÄISCHE PATENTSCHRIFT

(11) **EP 1 002 038 B1**
(45) Veröffentlichungstag und Bekanntmachung des Hinweises auf die Patenterteilung: **22.09.2004**
(21) Anmeldenummer: 98941397.6
(22) Anmeldetag: 21.07.1998
(51) Int. Cl.: C11D 3/37, C11D 3/20, C11D 1/83, A61K 7/50, A61K 7/06

(54) **WÄSSRIGE PERLGLANZKONZENTRATE**
AQUEOUS PEARLESCENT CONCENTRATES
CONCENTRES DE LUSTRE PERLAIRE AQUEUX

(30) Priorität: 30.07.1997 DE 19732708
(43) Veröffentlichungstag der Anmeldung: 24.05.2000
(73) Patentinhaber: Cognis Deutschland GmbH & Co. KG, 40589 Düsseldorf (DE)
(72) Erfinder: ANSMANN, Achim, D-40699 Erkrath (DE); BEHLER, Ansgar, D-46240 Bottrop (DE); KAWA, Rolf, D-40789 Monheim (DE); KREISIG, Annette, D-40229 Düsseldorf (DE)
(86) Internationale Anmeldenummer: PCT/EP1998/004580
(87) Internationale Veröffentlichungsnummer: WO 1999/006514

(56) Entgegenhaltungen:
- DE-A- 3 843 572
- DE-A- 4 103 551
- DE-A- 19 511 570

## Beschreibung

### Gebiet der Erfindung

Die Erfindung betrifft wäßrige Perlglanzkonzentrate mit einem Gehalt an ausgewählten Fettstoffen, Emulgatoren und gegebenenfalls Polyolen, ein Verfahren zu ihrer Herstellung, ein weiteres Verfahren zur Herstellung von perlglänzenden oberflächenaktiven Zubereitungen unter Verwendung der Konzentrate sowie die Verwendung der Fettstoffe als Perlglanzwachse.

### Stand der Technik

Der weich schimmernde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm. 75, 578 (1994).**

Der Stand der Technik kennt eine Vielzahl von Formulierungen, die oberflächenaktiven Mitteln den gewünschten Perlglanz verleihen. So sind beispielsweise aus den beiden Deutschen Patentanmeldungen **DE-A1 38 43 572** und **DE-A1 41 03 551** (Henkel) Perlglanzkonzentrate in Form fließfähiger wäßriger Dispersionen bekannt, die 15 bis 40 Gew.-% perlglänzender Komponenten, 5 bis 55 Gew.-% Emulgatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP-B1 0 181 773 und EP-B1 0 285 389** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP-A2 0 205 922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0 569 843** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP-A2 0 581 193** (Hoechst) sind ferner fließfähige, konservierungsmittelfreie Perlglanzdispersionen bekannt, die acylierte Polyglycolether, Betaine, Aniontenside und Glycerin enthalten. Schließlich wird in der Europäischen Patentanmeldung **EP-A1 0 684 302** (Th.Goldschmidt) die Verwendung von Polyglycerinestern als Kristallisationshilfsmittel für die Herstellung von Perlglanzkonzentraten vorgeschlagen.

Trotz der Vielzahl von Mitteln besteht im Markt ein ständiges Bedürfnis nach neuen Perlglanzwachsen, die sich gegenüber den Produkten des Stands der Technik auch bei verminderter Einsatzmenge durch einen brillanten Glanz auszeichnen, die die Mitverwendung kritischer Inhaltsstoffe wie beispielsweise von Siliconen zulassen, ohne daß die Stabilität der Formulierungen beeinträchtigt wird und die insbesondere in konzentrierter Form noch leicht beweglich und damit handhabbar sind. Die Aufgabe der vorliegenden Erfindung hat somit darin bestanden, neue Perlglanzkonzentrate mit dem geschilderten komplexen Anforderungsprofil zur Verfügung zu stellen.

### Beschreibung der Erfindung

Gegenstand der Erfindung sind wäßrige Perlglanzkonzentrate, enthaltend - bezogen auf den nicht-wäßrigen Anteil -
**(a)** 1 bis 99,1 Gew.-% Fettether der Formel (I)

   **R**^{**1**} **- (OC**_{**n**}**H**_{**2n**}**)**_{**x**} **- O - (C**_{**m**}**H**_{**2m**}**O)** _{**y**} **- R**^{**2**} **(I)**

   in der R¹ und R² unabhängig voneinander für Alkyl- und/ oder Alkenylreste mit 4 bis 24 Kohlenstoffatomen stehen, mit der Maßgabe, daß sie in Summe mindestens 28 Kohlenstoffatome enthalten und x und y unabhängig voneinander für Zahlen von 0 bis 10 stehen, mit der Maßgabe, daß die Summe aus x und y 1 bis 10 beträgt, sowie n und m unabhängig voneinander für Zahlen zwischen 2 und 4 stehen,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

Überraschenderweise wurde gefunden, daß die genannten langkettigen Fettether ausgezeichnete perlglänzende Eigenschaften besitzen und sich gegenüber den Produkten des Stands der Technik durch eine höhere Brillanz bei geringerer Einsatzmenge, besondere Feinteiligkeit und Lagerstabilität auszeichnen. Insbesondere überraschend war, daß die erfindungsgemäßen Fettether trotz der relativ hohen Schmelztemperatur sich leicht zu Perlglanzkonzentraten verarbeiten lassen, ohne daß ungelöste Wachsbestandteile anfallen. Die Perlglanzwachse sind in konzentrierter Form dünnflüssig und erlauben auch die Einarbeitung von problematischen Inhaltsstoffen wie beispielsweise Siliconen in kosmetische Zubereitungen.

### Fettether

Erfindungsgemäß werden als Komponente (a) Fettether der Formel (I) eingesetzt

**R**^{**1**} **- (OC**_{**n**}**H**_{**2n**}**)**_{**x**} **- O - (C**_{**m**}**H**_{**2m**}**O)** _{**y**} **- R**^{**2**} **(I)**

in der R¹ und R² unabhängig voneinander für Alkyl- und/ oder Alkenylreste mit 4 bis 24 Kohlenstoffatomen stehen, mit der Maßgabe, daß sie in Summe mindestens 28, vorzugsweise mindestens 32 bis 48 Kohlenstoffatome enthalten und x und y unabhängig voneinander für Zahlen von 0 bis 10 stehen, mit der Maßgabe, daß die Summe aus x und y 1 bis 10, vorzugsweise 2 bis 8 und insbesondere 4 bis 6 beträgt. n und m stehen unabhängig voneinander für Zahlen zwischen 2 und 3, vorzugsweise ist n gleich 2.

Fettether der genannten Art werden üblicherweise durch Kondensation der entsprechenden alkoxylierten Alkohole hergestellt, wobei als Alkohol insbesondere Fettalkohole eingesetzt werden können.

### Fettalkohole

Unter Fettalkoholen sind primäre aliphatische Alkohole der Formel (II) zu verstehen,

**R**^{**3**}**OH** **(II)**

in der R³ für einen aliphatischen, linearen oder verzweigten Kohlenwasserstoffrest mit 4 bis 24 Kohlenstoffatomen und 0 und/oder 1, 2 oder 3 Doppelbindungen steht.

Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Daneben sind auch Guerbetalkohole besonders geeignet.

Bevorzugt sind technische Fettalkohole wie beispielsweise Kokos-, Palm-, Palmkern- oder Talgfettalkohol sowie deren angehärtete und vollständig gehärtete Typen..

Um zu den erfindungsgemäßen Fettethern zu gelangen kann man beispielsweise Fettalkohole der vorgenannten Art alkoxylieren, vorzugsweise ethoxylieren und/ oder propoxylieren, insbesondere bevorzugt werden die Fettalkohole ethoxyliert. Die Alkoxylierung der Fettalkohole kann beispielsweise derart erfolgen, daß sie in einer Random-Polymerisation mit Ethylenoxid und Propylenoxid umgesetzt werden, man kann jedoch auch beispielsweise EO/PO-Blockpolymere herstellen. Beide Arten alkoxylierter Verbindungen sollen im Rahmen der vorliegenden Erfindung umfaßt sein. Bei der Alkoxylierung erhält man eine Homologenverteilungen, besonders bevorzugt werden alkoxylierte Fettalkohole mit enger Homologenverteilung zur Herstellung der erfindungsgemäßen Fettether eingesetzt. Bei anschließender saurer oder basischer Kondensation der entsprechenden alkoxylierten Fettalkohole gelangt man zu den erfindungsgemäß einzusetzenden Fettethern. Diese umfassen sowohl symmetrische als auch unsymmetrische Fettether.

Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.

### Emulgatoren

Die erfindungsgemäßen Perlglanzkonzentrate können als Emulgatoren **nichtionogene** Tenside aus mindestens einer der folgenden Gruppen enthalten:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat, Polyglycerindimerate oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(b9) Trialkylphosphate sowie Mono-, Di- und/oder Tri-PEG-alkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren **zwitterionische** Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind **ampholytische Tenside.** Unter ampholytischen Tensiden werden solche oberflächenaktiven Verbindungen verstanden, die außer einer C_{8/18}-Alkyloder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Daneben sind als Emulgatoren **anionische Tenside** geeignet wie beispielsweise Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizen-basis) und Alkyl(ether)phosphate. Sofern die anionischen Tenside Polyglycoletherketten enthalten, können diese eine konventionelle, vorzugsweise jedoch eine eingeengte Homologenverteilung aufweisen. Weiterhin kommen erfindungsgemäß als Emulgatoren auch **kationische Tenside** in Frage wie zum Beispiel quartäre Ammoniumverbindungen und Esterquats, vorzugsweise methyl-quaternierte Difettsäuretriethanolaminester-Salze, besonders bevorzugt sind quaternierte Fettsäuretrialkanolaminestersalze.

Die erfindungsgemäßen Perlglanzkonzentrate können die Emulgatoren in Mengen von 0,1 bis 90, vorzugsweise 5 bis 50 und insbesondere 10 bis 40 Gew.-% enthalten.

### Polyole

Polyole, die im Sinne der Erfindung als Komponente (c) in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die erfindungsgemäßen Perlglanzkonzentrate können die Polyole, vorzugsweise Glycerin, Propylenglycol, Butylenglycol, Hexylenglycol sowie Polyethylenglycole mit einem durchschnittlichen Molekulargewicht im Bereich von 100 bis 1.0000 in Mengen von 0,1 bis 40, vorzugsweise 0,5 bis 15 und insbesondere 1 bis 5 Gew.-% enthalten.

### Herstellverfahren

In einer bevorzugten Ausführungsform, die ebenfalls Gegenstand der Erfindung ist, erfolgt die Herstellung der Perlglanzkonzentrate, indem man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt. Ferner ist es möglich, eine konzentrierte wäßrige (Anion-)Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel, wie etwa Hydroxypropylcellulosen, Xanthan Gum oder Polymeren vom Carbomer-Typ durchzuführen.

### Gewerbliche Anwendbarkeit

Die erfindungsgemäßen Perlglanzkonzentrate eignen sich zur Einstellung einer Trübung in oberflächenaktiven Zubereitungen wie beispielsweise Haarshampoos oder manuellen Geschirrspülmitteln. Ein weiterer Gegenstand der Erfindung betrifft daher ein Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C die Perlglanzkonzentrate in einer Menge von 0,5 bis 40, vorzugsweise 1 bis 20 Gew.-% der Zubereitung zusetzt und unter Rühren darin verteilt.

### Tenside

Die oberflächenaktiven Zubereitungen, die in der Regel einen nicht-wäßrigen Anteil im Bereich von 1 bis 50 und vorzugsweise 5 bis 35 Gew.-% aufweisen, können nichtionische, anionische, kationische und/oder amphotere bzw. amphotere Tenside enthalten, deren Anteil an den Mitteln üblicherweise bei etwa 50 bis 99 und vorzugsweise 70 bis 90 Gew.-% beträgt. Typische Beispiele für anionische **Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Die gleichen Tenside können auch unmittelbar zur Herstellung der Perlglanzkonzentrate eingesetzt werden, die anionischen Tenside eignen sich auch als Emulgatoren.

### Hilfs- und Zusatzstoffe

Die oberflächenaktiven Zubereitungen, denen die erfindungsgemäßen Perlglanzkonzentrate zugesetzt werden, können weitere Hilfs- und Zusatzstoffe enthalten, wie beispielsweise Ölkörper, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farbstoffe, Parfümöle, anorganische Schichtsilikate wie beispielsweise Montmorillonite, Hautbräuningsmittel wie beispielsweise Dihydroxyaceton sowie Pigmente, insbesondere Titandioxid und Zinkoxid.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht wie beispielsweise FinsolvTN.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quatemierte VinylpyrrolidonNinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldlethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quatemierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Im Sinne der Erfindung können neben den Fettstoffen auch weitere bekannte **Perlglanzwachse** wie insbesondere Mono- und Difettsäureester von Polyalkylenglycolen, Partial- und Triglyceride sowie Ester von Fettalkoholen mit mehrwertigen Carbonsäuren bzw. Hydroxycarbonsäuren eingesetzt werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder die bereits oben genannten Polyole eingesetzt werden. Als **Konsevierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81.106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein letzter Gegenstand der Erfindung betrifft schließlich die Verwendung der genannten Fettether gemäß Formel (I) als Perlglanzwachse zur Herstellung von oberflächenaktiven Zubereitungen.

### Beispiele

Die Vergleichsmischung R5 wurde 14 Tage bei 40°C gelagert und die Viskosität nach der Brookfield-Methode in einem RVT-Viskosimeter (23°C, 10 Upm, Spindel 5) bestimmt. Anschließend wurde eine wäßrige Haarshampooformulierung durch Vermischen der Einsatzstoffe bei 20°C zubereitet, die 2 g der Periglanzkonzentrate R5, 15 g Kokosfettalkohol+2EO-sulfat-Natriumsalz, 3 g Dimethylpolysiloxan, 5 g Kokosalkylglucosid und 1,5 g eines Esterquats (Wasser ad 100 Gew.-%) enthielt. Die Feinteiligkeit der Perlglanzkristalle in den Haarshampoos wurde unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Zusammensetzung und Ergebnisse sind in Tabelle 1 zusammengefaßt; alle Mengenangaben verstehen sich als Gew.-%

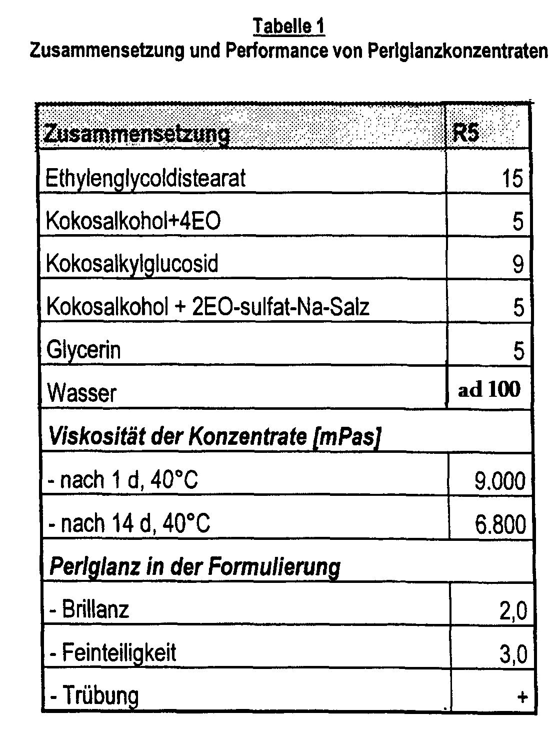

**Tabelle 2**

| **Formulierungsbeispiele für perlglänzende oberflächenaktive Mittel** | | | | | |
|---|---|---|---|---|---|
| | **Duschgel** | **Konditioniershampoo** | **Waschlotion** | **Schaumbad** | **Pflegeshampoo** |
| **Texapon NSO** Sodium Laureth Sulfate | 40 | 25 | 28 | 20 | 10 |
| **Plantacare 1200** Lauryl Glucoside | 5 | 10 | 12 | 20 | 5 |
| **Dehyton K** Cocamidopropyl Betaine | 10 | - | 15 | - | 8 |
| **Lamepon S** Potassium Cocoyl Hydrolyzed Collagen | - | - | - | 20 | 4,5 |
| **Lanette O** Cetearyl Alcohol | - | 1 | - | - | 1 |
| **Lamesoft PO 65** Coco-Glucoside, Glyceryl Oleate | 2,5 | - | 2 | 2 | - |
| **Dehyquart A** Cetrimonium Chloride | - | 2 | - | - | 2,5 |
| Dimethylpolysiloxan | - | 1,5 | - | - | 0,7 |
| **Fettether der Formel (I)** | - | - | 1,3 | - | 1 |
| **Konzentrat gem. Erfindung** | 5 | 7,5 | - | 8 | - |
| Natriumchlorid | 1 | 1 | 1 | 1 | 1,5 |
| Wasser | ad 100 | ad 100 | ad 100 | ad 100 | ad 100 |
| Die Mengenangaben sind Gew.% bezogen auf die gesamte Formulierung. Daneben enthalten alle Formulierungen noch Parfüm, Konservierungsmittel, Farbstoffe in untergeordneter Menge | | | | | |

## Patentansprüche

1. Wäßrige Perlglanzkonzentrate, enthaltend - bezogen auf den nicht-wäßrigen Anteil -
(a) 1 bis 99,1 Gew.-% Fettether der Formel (I)
**R**^{**1**} **- (OC**_{**n**}**H**_{**2n**}**)**_{**x**} **- O - (C**_{**m**}**H**_{**2m**}**O)** _{**y**}**- R**^{**2**} **(I)**
in der R¹ und R² unabhängig voneinander für Alkyl- und/ oder Alkenylreste mit 4 bis 24 Kohlenstoffatomen stehen, mit der Maßgabe, daß sie in Summe mindestens 28 Kohlenstoffatome enthalten und x und y unabhängig voneinander für Zahlen von 0 bis 10 stehen, mit der Maßgabe, daß die Summe aus x und y 1 bis 10 beträgt, sowie n und m unabhängig voneinander für Zahlen zwischen 2 und 4 stehen,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

2. Perlglanzkonzentrate nach Anspruch 1, **dadurch gekennzeichnet, daß** sie als Komponente (a) Fettether der Formel **(I)** enthalten, in der R¹ und R² unabhängig voneinander für Alkyl- und/oder Alkenylreste mit 12 bis 22 Kohlenstoffatomen stehen, mit der Maßgabe, daß sie in Summe mindestens 28 Kohlenstoffatome aufweisen.

3. Perlglanzkonzentrate nach den Ansprüchen 1 und 2, **dadurch gekennzeichnet, daß** sie als Komponente (a) Fettether der Formel **(I)** enthalten, in der n und m gleich 2 sind.

4. Perlglanzkonzentrate nach den Ansprüchen 1 bis 3, **dadurch gekennzeichnet, daß** sie als Komponente (a) Fettether der Formel **(I)** enthalten, in der x und y gleich sind und für Zahlen von 2 bis 8 stehen.

5. Perlglanzkonzentrate nach den Ansprüchen 1 bis 4, **dadurch gekennzeichnet, daß** sie als Komponente (b) Emulgatoren enthalten, die ausgewählt sind aus der Gruppe, die gebildet wird von :
(b1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
(b2) C_{12/18}Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat, Polyglycerindimerate oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(b9) Trialkylphosphate sowie Mono-, Di- und/oder Tri-PEG-alkylphosphate;
(b10) Wollwachsalkohole;
(b11) Polysiloxan-Polyalkyl-Polyether-Copolymere bzw. entsprechende Derivate;
(b12) Mischester, die Kondensationsprodukte aus einem Pentaerythrit-difettsäureester und einem Citronensäure-di-fettalkoholester im Molverhältnis 1:1 darstellen, wobei die Veresterung der Citronensäure unter Verwendung von wäßriger Citronensäure erfolgt und/oder Mischester von Fettsäuren mit 6 bis 22 Kohlenstoffatomen, Methylglucose und Polyolen, vorzugsweise Glycerin sowie
(b13) Polyalkylenglycole.

6. Perlglanzkonzentrate nach den Ansprüchen 1 bis 5, **dadurch gekennzeichnet, daß** sie als Komponente (b) Emulgatoren vom Typ der zwitterionischen Tenside und/oder Esterquats enthalten.

7. Perlglanzkonzentrate nach den Ansprüchen 1 bis 6, **dadurch gekennzeichnet, daß** sie als Komponente (c) 0,1 bis 40 Gew.-% Glycerin, 1,2-Propylenglycol, Butylenglycol, Hexylenglycol und/oder Polyethylenglycole mit einem durchschnittlichen Molekulargewicht im Bereich von 100 bis 1.000 Dalton enthalten.

8. Verfahren zur Herstellung von Perlglanzkonzentraten nach Anspruch 1, **dadurch gekennzeichnet, daß** man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt.

9. Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C Perlglanzkonzentrate nach den Ansprüchen 1 bis 8 in einer Menge von 0,5 bis 40 Gew.-% der Zubereitung zusetzt und unter Rühren darin verteilt.

10. Verwendung von Fettethem gemäß Formel (I) als Perlglanzwachse zur Herstellung von oberflächenaktiven Zubereitungen.

## Claims

1. Aqueous pearlizing concentrates containing - based on the nonaqueous component -
(a) 1 to 99.1 % by weight of fatty ethers corresponding to formula (I):
**R**^{**1**}**-(OC**_{**n**}**H**_{**2n**}**)**_{**x**} **- O- (C**_{**m**}**H**_{**2m**}**O)**_{**y**} **- R**^{**2**} **(I)**
in which R¹ and R² independently of one another represent alkyl and/or alkenyl groups containing 4 to 24 carbon atoms, with the proviso that, in all, they contain at least 28 carbon atoms, and x and y independently of one another are numbers of 0 to 10, with the proviso that the sum of x and y is 1 to 10, and n and m independently of one another are numbers of 2 to 4,
(b) 0.1 to 90% by weight of anionic, nonionic, cationic, ampholytic and/or zwitterionic emulsifiers and
(c) 0 to 40% by weight of polyols,
with the proviso that the quantities shown add up to 100% by weight.

2. Pearlizing concentrates as claimed in claim 1, **characterized in that** they contain as component (a) fatty ethers corresponding to formula (I) in which R¹ and R² independently of one another represent alkyl and/or alkenyl groups containing 12 to 22 carbon atoms, with the proviso that, in all, they contain at least 28 carbon atoms.

3. Pearlizing concentrates as claimed in claims 1 and 2, **characterized in that** they contain as component (a) fatty ethers corresponding to formula (I) in which n and m = 2.

4. Pearlizing concentrates as claimed in claims 1 to 3, **characterized in that** they contain as component (a) fatty ethers corresponding to formula (I) in which x and y are the same and stand for numers of 2 to 8.

5. Pearlizing concentrates as claimed in claims 1 to 4, **characterized in that** they contain as component (b) emulsifiers selected from the group consisting of:
(b1) products of the addition of 2 to 30 mol ethylene oxide and/or 0 to 5 mol propylene oxide onto linear fatty alcohols containing 8 to 22 carbon atoms, onto fatty acids containing 12 to 22 carbon atoms, onto alkylphenols containing 8 to 15 carbon atoms in the alkyl group and onto triglycerides;
(b2) C_{12/18} fatty acid monoesters and diesters of addiion products of 1 to 30 mol ethylene oxide onto glycerol;
(b3) glycerol monoesters and diesters and sorbitan monoesters and diesters of saturated and unsaturated fatty acids containing 6 to 22 carbon atoms and ethylene oxide adducts thereof;
(b4) alkyl mono- and oligoglycosides containing 8 to 22 carbon atoms in the alkyl group and ethoxylated analogs thereof;
(b5) products of the addition of 15 to 60 mol ethylene oxide onto castor oil and/or hydrogenated castor oil;
(b6) polyol esters and, in particular, polyglycerol esters such as, for example, polyglycerol polyricinoleate, polyglycerol dimerates or polyglycerol poly-12-hydroxystearate. Mixtures of compounds from several of these classes are also suitable;
(b7) products of the addition of 2 to 15 mol ethylene oxide onto castor oil and/or hydrogenated castor oil;
(b8) partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
(b9) trialkyl phosphates and mono-, di- and/or tri-PEG-alkyl phosphates;
(b10) wool wax alcohols;
(b11) polysiloxane/polyalkyl polyether copolymers and corresponding derivatives;
(b12) mixed esters in the form of condensation products of a pentaerythritol difatty acid ester and a citric acid difatty alcohol ester in a molar ratio of 1:1, the esterification of the citric acid with fatty alcohol being carried out using aqueous citric acid, and /or mixed esters of fatty acids containing 6 to 22 carbon atoms, methyl glucose and polyols, preferably glycerol or polyglycerol, and
(b13) polyalkylene glycols.

6. Pearlizing concentrates as claimed in claims 1 to 5, **characterized in that** they contain emulsifiers of the zwitterionic surfactant and/or esterquat type as component (b).

7. Pearlizing concentrates as claimed in claims 1 to 6, **characterized in that** they contain 0.1 to 40% by weight of glycerol, 1,2-propylene glycol, butylene glycol, hexylene glycol and/or polyethylene glycols with an average molecular weight of 100 to 1,000 dalton as component (c).

8. A process for the production of the pearlizing concentrates claimed in claim 1, **characterized in that** a mixture of components (a), (b) and (c) is prepared, heated to a temperature 1 to 30°C above the melting point of the mixture, mixed with the necessary quantity of water having substantially the same temperature and then cooled to room temperature.

9. A process for the production of opaque and pearlescent liquid water-containing preparations of water-soluble surface-active substances, in which the pearlizing concentrates claimed in claims 1 to 8 are added to the clear aqueous preparations at 0 to 40°C in a quantity of 0.5 to 40% by weight, based on the preparation, and distributed therein by stirring.

10. The use of the fatty ethers corresponding to formula (I) as pearlizing waxes for the production of surface-active formulations.

## Revendications

1. Concentrés nacrés aqueux contenant - par rapport à la fraction non aqueuse -
(a) 1 à 99,1 % en poids d'éther gras de formule (I)
R¹ - (OCₙH₂ₙ)ₓ - O - (CₘH₂ₘO) y - R² **(I)**
dans laquelle R¹ et R² représentent, indépendamment l'un de l'autre, des radicaux alkyle et/ ou alcényle portant 4 à 24 atomes de carbone, étant précisé qu'ils contiennent, au total, au moins 28 atomes de carbone, x et y représentent, indépendamment l'un de l'autre, des nombres de 0 à 10, étant précisé que le total de x et y s'élève de 1 à 10, et n et m représentent, indépendamment l'un de l'autre, des nombres compris entre 2 et 4,
(b) 0,1 à 90 % en poids d'émulsifiants anioniques, non ioniques, cationiques, ampholytiques et/ou zwitterioniques ainsi que
(c) 0 à 40 % en poids de polyols,
étant précisé que les quantités indiquées font un total de 100 % en poids.

2. Concentrés nacrés selon la revendication 1,
**caractérisés en ce qu'**
ils contiennent, en tant que composant (a) des éthers gras de formule (I), dans laquelle R¹ et R² représentent, indépendamment l'un de l'autre, des radicaux alkyle et/ou alcényle portant 12 à 22 atomes de carbone étant précisé qu'ils présentent, au total, au moins 28 atomes de carbone.

3. Concentrés nacrés selon les revendications 1 et 2,
**caractérisés en ce qu'**
ils contiennent, en tant que composant (a) des éthers gras de formule (I) dans laquelle n et m sont égaux à 2.

4. Concentrés nacrés selon les revendications 1 à 3,
**caractérisés en ce qu'**
ils contiennent, en tant que composant (a) des éthers gras de formule (I) dans laquelle x et y sont égaux et représentent des nombres de 2 à 8.

5. Concentrés nacrés selon les revendications 1 à 4,
**caractérisés en ce qu'**
ils contiennent, en tant que composant (b) des émulsifiants choisis dans le groupe formé par :
(b1) des produits d'addition de 2 à 30 moles d'oxyde d'éthylène et/ou de 0 à 5 moles d'oxyde de propylène sur des alcools gras linéaires portant 8 à 22 atomes de C, sur des acides gras portant 12 à 22 atomes de C, sur des phénols d'alkyle portant 8 à 15 atomes de C dans le groupe alkyle et sur des triglycérides ;
(b2) des mono et diesters d'acides gras en C_{12/18} de produits d'addition de 1 à 30 moles d'oxyde d'éthylène sur la glycérine ;
(b3) des mono et diesters de glycérine et des mono et diesters de sorbitane d'acides gras saturés et insaturés portant 6 à 22 atomes de carbone et leurs produits d'addition d'oxyde d'éthylène ;
(b4) des mono et oligoglycosides d'alkyle portant 8 à 22 atomes de carbone dans le radical alkyle et leurs analogues éthoxylés ;
(b5) des produits d'addition de 15 à 60 moles d'oxyde d'éthylène sur l'huile de ricin et/ou l'huile de ricin durcie ;
(b6) des esters de polyol et, en particulier, des esters de polyglycérine comme, par exemple, le polyricinoléate de polyglycérine, les dimérates de polyglycérine ou le poly-12-hydroxystéarate de polyglycérine ; conviennent également les mélanges de composés constitués de plusieurs de ces classes de substances ;
(b7) des produits d'addition de 2 à 15 moles d'oxyde d'éthylène sur l'huile de ricin et/ou l'huile de ricin durcie ;
(b8) des esters partiels à base d'acides gras en C_{6/22} linéaires, ramifiés, insaturés ou saturés, de l'acide ricinolique ainsi que de l'acide 12-hydroxystéarique et de la glycérine, de la polyglycérine, de la pentaérythrite, de la dipentaérythrite, des alcools de sucre (le sorbite par exemple), des glucosides d'alkyle (par exemple glucoside de méthyle, glucoside de butyle, glucoside de lauryle) ainsi que des polyglucosides (par exemple la cellulose) ;
(b9) des phosphates de trialkyle ainsi que des phosphates de mono-, di- et/ou tri-PEG-alkyle ;
(b10) des alcools de cire de laine;
(b11) des copolymères polysiloxane-polyalkyle-polyéther ou des dérivés correspondants ;
(b12) des esters mixtes qui constituent les produits de condensation d'un diester d'acide gras de pentaérythrite et d'un diester d'alcool gras de l'acide citrique dans un rapport molaire de 1:1, l'estérification de l'acide citrique étant réalisée en utilisant de l'acide citrique aqueux et/ou des esters mixtes d'acides gras portant 6 à 22 atomes de carbone, du méthylglucose et des polyols, de préférence de la glycérine ainsi que
(b13) des polyalkylène glycols.

6. Concentrés nacrés selon les revendications 1 à 5,
**caractérisés en ce qu'**
ils contiennent, en tant que composant (b) des émulsifiants du type des tensioactifs zwitterioniques et/ou des esters d'ammonium quaternaire.

7. Concentrés nacrés selon les revendications 1 à 6,
**caractérisés en ce qu'**
ils contiennent, en tant que composant (c) 0,1 à 40 % en poids de glycérine, de 1,2-propylène glycol, de butylène glycol, d'hexylène glycol et/ou de polyéthylène glycols ayant une masse molaire moyenne de l'ordre de 100 à 1.000 Dalton.

8. Procédé de fabrication de concentrés nacrés selon la revendication 1,
**caractérisé en ce qu'**
on fabrique un mélange à partir des composants (a), (b) et (c), on le chauffe à une température supérieure de 1 à 30°C au point de fusion du mélange, on le mélange à la quantité nécessaire d'eau à approximativement la même température, puis on le refroidit à la température ambiante.

9. Procédé de fabrication de préparations aqueuses liquides turbides et nacrées de substances tensioactives hydrosolubles, selon lequel on ajoute aux préparations aqueuses claires, à 0 à 40°C, des concentrés nacrés selon les revendications 1 à 8 dans une quantité de 0,5 à 40 % en poids de la préparation et on les y répartit sous agitation.

10. Utilisation d'éthers gras selon la formule (I) en tant que cires nacrées pour la fabrication de préparations tensioactives.
